Europäisches Patentamt

European Patent Office

Office européen des brevets

(19)

(11) Veröffentlichungsnummer: **0 054 674**
**B1**

(12) **EUROPÄISCHE PATENTSCHRIFT**

(45) Veröffentlichungstag der Patentschrift:
04.04.84

(21) Anmeldenummer: 81108693.3

(22) Anmeldetag: 22.10.81

(51) Int. Cl.³: **B 01 J 27/10,** B 01 J 23/78,
C 07 C 17/15

(54) **Kupfer- und Alkalimetalle enthaltender Trägerkatalysator.**

(30) Priorität: 10.12.80 DE 3046407

(43) Veröffentlichungstag der Anmeldung:
30.06.82 Patentblatt 82/26

(45) Bekanntmachung des Hinweises auf die Patenterteilung:
04.04.84 Patentblatt 84/14

(84) Benannte Vertragsstaaten:
BE DE FR GB IT NL

(56) Entgegenhaltungen:
DE - A - 2 651 974
DE - B - 1 284 411
DE - B - 2 356 549
FR - A - 2 409 793
US - A - 3 148 222

(73) Patentinhaber: BASF Aktiengesellschaft,
Carl-Bosch-Strasse 38, D-6700 Ludwigshafen (DE)

(72) Erfinder: Laurer, Peter Rudolf, Dr., Freinsheimer
Strasse 33, D-6700 Ludwigshafen (DE)
Erfinder: Krome, Gerd, Dr., Am Wingertsberg 28,
D-6719 Weisenheim (DE)
Erfinder: Cordemans, Luc, Dr., Kalmthoutsestw. 54-2,
B-2080 Kapellen (BE)
Erfinder: Seifert, Reinhard, Dr., Kerschensteiner
Strasse 13, D-6700 Ludwigshafen (DE)
Erfinder: Danz, Eckehard, Dr.,
Hermann-Hesse-Strasse 42, D-6700 Ludwigshafen (DE)

## Kupfer- und Alkalimetalle enthaltender Trägerkatalysator

Die Erfindung betrifft einen Kupfer- und Alkalimetalle enthaltenden Trägerkatalysator, der zur Herstellung von 1,2-Dichlorethan durch Oxichlorierung von Ethylen geeignet ist.

Zum Stand der Technik nennen wir:

(1)  GB-PS 1 104 666
(2)  DE-PS 2 356 549

Die Oxichlorierung von Ethylen ist ein bekanntes, großtechnisch ausgeübtes Verfahren. Es sind Verfahren bekannt, bei denen die Oxichlorierung nur in einem Reaktor durchgeführt wird. Dabei wird der erforderliche Sauerstoff gemeinsam mit HCl und Ethylen über eine einzige, durch geeignete Wärmeabführung möglichst isotherm betriebene Katalysatorschicht geleitet. In (1) wird beschrieben, die Umsetzung des Ethylens mit Chlorwasserstoff und Sauerstoff in mehreren hintereinander angeordneten Reaktoren durchzuführen, wobei der Sauerstoff, in mehrere Ströme aufgeteilt, jedem Reaktor gesondert zugeführt wird. Die Reaktoren sind mit einem Katalysator beschickt, der im wesentlichen Kupferchlorid oder das Oxichlorid des Kupfers auf einem Träger niedergeschlagen enthält. Eine besondere Ausführungsform des bekannten Verfahrens besteht darin, daß die Reaktoren in Zonen unterschiedlicher Katalysatoraktivität aufgeteilt sind.

Die Katalysatoren für den genannten Verwendungszweck können zusätzlich zu Kupfer-chlorid bzw. -oxichlorid noch andere Promotoren enthalten. So sind insbesondere die Chloride der Alkalien, der Erdalkalimetalle, des Silbers, des Zinks sowie die der seltenen Erde, insbesondere die von Cer als geeignet in der Literatur beschrieben.

Die bekannten Katalysatoren besitzen jedoch den Nachteil, daß sie bei hoher Aktivität nur geringe Selektivitäten aufweisen. Es bestand daher die Aufgabe, die Nachteile der bekannten Katalysatoren zu beheben. Die Lösung der Aufgabe gelingt mit Hilfe eines Katalysators gemäß Patentanspruch 1.

Der erfindungsgemäße Katalysator enthält Kupfer in Mengen von 1 bis 12 Gew.-%, bezogen auf den Gesamtkatalysator und berechnet als Metall. Das Kupfer wird bei der Herstellung des Katalysators meist in Form des Kupferchlorids bzw. des Kupferoxichlorids eingebracht. Vorzugsweise werden Kupfergehalte von 1,5 bis 9 Gew.-%, berechnet als metallisches Kupfer und bezogen auf den Gesamtkatalysator, angewendet.

Der erfindungsgemäße Katalysator enthält zusätzlich einen Gehalt von 0,3 bis 9 Gew.-% der Alkalimetalle Kalium, Lithium und Natrium. Es wird bevorzugt, 0,3 bis 3 Gew.-% der 3 Alkalimetalle insgesamt anzuwenden, besonders bevorzugt ist ein Gehalt von 0,5 bis 1,5 Gew-% der 3 Alkalimetalle. Jedes der Alkalimetalle wird in einem Anteil von je 0,1 bis 3 Gew.-% angewendet. Besonders bevorzugt werden 0,3 bis 1,0 Gew.-% von jedem der Alkalimetalle angewendet (alle Angaben beziehen sich auf den Gesamtkatalysator, die Alkalimetalle werden berechnet als Metall).

Die Alkalimetalle werden bei der Herstellung des Katalysators bevorzugt in Form der Chloride d. h. als Kaliumchlorid, Natriumchlorid und Lithiumchlorid eingebracht und sind in dieser Form in dem Katalysator enthalten.

Im Rahmen der vorliegenden Erfindung ist dem Fachmann bekannt, daß er Katalysatoren geringerer Aktivität dadurch herstellen kann, daß er das Gewichtsverhältnis Summe Alkalimetalle zu Kupferchlorid auf Werte von bis zu 1 : 1 erhöht, um für die in (1) beschriebene Eingangszone mit hohem Sauerstoffpartialdruck geeignete Katalysatoren herzustellen. Das Gewichtsverhältnis der Summe Alkalimetalle : Kupfer kann in weiten Grenzen von 0,05 bis 1,0 gewählt werden.

Der Katalysator enthält außer den Promotoren Kupfer und den Alkalimetallen einen Träger, dessen Menge den Gesamtkatalysator zu 100% ergänzt. Als Träger für den erfindungsgemäßen Katalysator wird bevorzugt aktives Aluminiumoxid. Insbesondere wird $\gamma$-$Al_2O_3$ als Aluminiumoxidmodifikation bevorzugt. Es sind jedoch auch Trägermaterialien aus den sogenannten Übergangsoxiden des $Al_2O_3$ sowie Mischungen aus Aluminiumoxiden und Kieselsäure sowie Aluminiumsilikaten geeignet. Der Katalysatorträger ist nach den dem Fachmann bekannten Zusammenhängen so herzustellen, daß er spezifische Oberflächen, gemessen nach BET, im Bereich von 80 bis 300 $m^2$/g bei Porenvolumina im Bereich von 0,4 bis 1,0 $cm^3$/g aufweist. Die Auswahl eines geeigneten Trägermaterials für die anschließende Herstellung des Katalysators ist dem Fachmann bekannt.

Die Herstellung des Katalysators erfolgt bevorzugt durch Tränken des Trägers mit einer wäßrigen Lösung, die Kupfer(II)-Salze, Chlorwasserstoff und die Chloride der Alkalimetalle in den entsprechenden Konzentrationen enthält. Abhängig von dem gewünschten Gehalt an Kupfer und Alkalimetallen kann die Tränkung des Trägers in einer einzigen Stufe durchgeführt werden. Dies ist auch die zweckmäßigste und wirtschaftlichste Methode. Es kann jedoch Fälle geben, bei denen eine Einfachtränkung nicht ausreicht, um die gewünschte Menge an Metallen auf den Träger aufzubringen. Die Maßnahmen, die der Fachmann zu ergreifen hat, um eine gewünschte Menge an Metallen auf einen Träger aufzubringen, sind bekannt. Die Tränkung des Katalysatorträgers bzw. die anschließende Behandlung der getränkten Formlinge ist in (2), Spalte 3, Zeilen 37 bis 67 eingehend beschrieben.

Bezüglich der Aktivität spielt auch die gewählte Form des Trägers eine gewisse Rolle. Als Trägerformen kommen Tabletten, Kugeln oder Ringe in Betracht. Besonders bevorzugt werden Ringe, die je nach dem gewünschten Verwendungszweck bzw. dem Einsatzort im Bereich der Abmessungen von

Da  5 – 12 mm  (Da  =  Außendurchmesser)
Di  3 –  8 mm  (Di  =  Innendurchmesser)
H   3 – 12 mm  (H   =  Höhe)

liegen.

Für die Durchführung der Beispiele und Vergleichsversuche I und II wurden die nachfolgend beschriebenen Katalysatoren A, B und C verwendet:

Katalysator A entsprach dem Katalysator des Beispiels von (2).

Katalysator B wurde entsprechend der Lehre (2) hergestellt, wobei aber abweichend von (2) zusätzlich Natriumchlorid verwendet wurde (nicht erfindungsgemäß, nicht Stand der Technik).

Katalysator C ist ein erfindungsgemäßer Katalysator, nach der Lehre in (2) unter Verwendung der Alkalimetallchloride, des Kaliums, des Natriums und des Lithiums hergestellt.

Für die Herstellung der genannten 3 Katalysatoren A, B und C wurden in allen Fällen zylindrische Tabletten mit den Abmessungen 5·5 mm aus $\alpha$-$Al_2O_3$ mit einer spezifischen Oberfläche von 150 m²/g und einer Porosität (Porenvolumen) von 0,75 cm³/g verwendet. Die Zusammensetzung der Katalysatoren ist nachstehend wiedergegeben. Alle Angaben in Gew.-%, (Rest, Träger).

|   | % Cu | % K | % Na | %Li |
|---|---|---|---|---|
| A | 7,5 | 0,77 | — | — |
| B | 7,5 | 0,76 | 0,87 | — |
| C | 6,9 | 0,74 | 0,90 | 0,87 |

Die Erfindung wird anhand der nachstehend beschriebenen Beispiele und Vergleichsversuche erläutert. Alle darin angegebenen Teile und Prozente beziehen sich, sofern nichts anderes vermerkt ist, auf das Gewicht.

### Beispiel 1 und Vergleichsversuch I und II

In den nachfolgend beschriebenen Versuchen wurden die Katalysatoren A, B und C in einem isotherm betriebenen Röhrenreaktor, bei einer Temperatur von 250°C für die Synthese von 1,2-Dichlorethan getestet. Das zur Umsetzung verwendete Gas enthielt 6,1 Volumenprozent HCl, 2,86 Volumenprozent $C_2H_4$ und 1,71 Volumenprozent $O_2$, der Rest war Stickstoff, der zugesetzt wurde, um Überhitzungen zu vermeiden.

Im Abgas des Reaktors wurde die Summe von CO und $CO_2$ in Volumenprozent bestimmt. Ebenfalls wurde bestimmt die Summe der gebildeten Chlorkohlenwasserstoffe in Gew.-%, sowie der Ethylenumsatz.

Der Ethylenumsatz ist ein Maß für die Aktivität des Katalysators; die gebildete Menge an CO und $CO_2$, herrührend von der Oxidation von Ethylen, kann als Maß für die Selektivität des Katalysators angesehen werden; dasselbe gilt auch für die Summe der chlorierten Kohlenwasserstoffe, die vom Wertprodukt durch Destillation abgetrennt bzw. aus dem Abgas durch Kondensation bei tiefen Temperaturen abgeschieden werden.

In Beispiel 1 (vgl. Katalysator C) und den Vergleichsversuchen I und II (vgl. Katalysator A bzw. B) wurden folgende Werte gefunden:

|  | Beispiel/Vergleichsweise | | |
|---|---|---|---|
|  | I | II | 1 |
|  | Katalysator | | |
|  | A | B | C |
| Oxidation (Vol.-% CO + CO₂) | 0,263 | 0,059 | 0,036 |
| Chlorkohlenwasserstoffe (Gew.-%) | 0,707 | 0,402 | 0,307 |
| Ethylenumsatz | 87,8 | 88,8 | 88,1 |

3

Ein Vergleich der Ergebnisse zeigt, daß bei etwa gleicher Aktivität der erfindungsgemäße Katalysator C gegenüber dem Katalysator A vom Stand der Technik eine deutlich verbesserte Selektivität aufweist. Dasselbe gilt beim Vergleich mit dem Katalysator B, der nicht zum Stand der Technik zu rechnen ist.

## Patentansprüche

1. Kupfer- und Alkalimetalle enthaltender Trägerkatalysator, dadurch gekennzeichnet, daß dieser einen Gehalt
an Kupfer von 1,0 bis 12 Gew.-%, bezogen auf den Gesamtkatalysator und berechnet als Metall, und
an den drei Alkalimetallen Kalium und Lithium und Natrium von insgesamt 0,3 bis 9 Gew.-%, bezogen auf den Gesamtkatalysator und berechnet als Metall, aufweist.

2. Verwendung des Katalysators gemäß Anspruch 1, zur Herstellung von 1,2-Dichlorethan durch Oxichlorierung von Ethan.

## Claims

1. A supported catalyst containing copper and alkali metals, wherein the content of copper is from 1.0 to 12% by weight, based on total catalyst and calculated as metal, and the total content of the three alkali metals potassium, lithium and sodium is from 0.3 to 9% by weight, based on total catalyst and calculated as metal.

2. The use of a catalyst as claimed in claim 1 for the production of 1,2-dichloroethane by oxychlorination of ethane.

## Revendications

1. Catalyseur fixé sur support, contenant du cuivre et des métaux alcalins, caractérisé en ce qu'il présente une teneur en cuivre de 1,0 à 12% en poids, calculée en tant que métal et par rapport au catalyseur total et une teneur en les trois métaux alcalins potassium, lithium et sodium de 0,3 à 9% en poids au total, calculée en tant que métaux et par rapport au catalyseur total.

2. Utilisation du catalyseur selon la revendication 1 pour la préparation de 1,2-dichloréthane par oxychloration d'éthane.